# EUROPÄISCHE PATENTANMELDUNG

(11) **EP 1 020 165 A2**
(43) Veröffentlichungstag der Anmeldung: **19.07.2000**
(21) Anmeldenummer: 99125713.0
(22) Anmeldetag: 23.12.1999
(51) Int. Cl.: A61F 2/04

(54) **Implantierbare künstliche Blase**

(30) Priorität: 13.01.1999 DE 19900940
(71) Anmelder: CareMed Medical Produkte Aktiengesellschaft, 01109 Dresden (DE)
(72) Erfinder: Gerlach, Roland, Dr.-Ing., 34302 Guxhagen (DE); Hannappel, Josef, Prof. Dr., 50259 Pulheim (DE); Reuter, Jürgen, Dipl.-Ing., 36211 Alheim (DE); Rohrmann, Dorothea, Priv.-Doz. Dr., 52379 Langerwehe (DE)
(74) Vertreter: Dallmeyer, Georg, Dipl.-Ing.

(57) **Zusammenfassung**

Die Blase besteht aus zwei Kammern 10,11, welche aus rückstellfähigem elastischem Material hergestellt sind. Zwischen beiden Kammern (10,11) befindet sich ein Rückschlagventil (23), das in Durchlaßrichtung vorgespannt ist und Flüssigkeit erst durchläßt, wenn die Druckdifferenz einen Grenzwert übersteigt. Die von der Niere (19) kommende Flüssigkeit gelangt zuerst in die erste Kammer (10) und wenn sich darin eine Flüssigkeitsmenge angesammelt hat durch das Ventil (23) in die zweite Kammer (11). Die Kammern (10,11) sind in einer 8-förmigen Konfiguration angeordnet. Die Blase hat eine flache Bauweise und ermöglicht eine günstige anatomische Anpassung an den Implantationsbereich zwischen Rippen und Beckenschaufel. Außerdem wird ein über den Füllgrad relativ gleichmäßig verlaufender Saugdruck erzeugt.

## Beschreibung

Die Erfindung betrifft eine implantierbare künstliche Blase, die einem Patienten subkutan eingepflanzt wird, um den aus den Nieren abgeschiedenen Urin aufzunehmen.

Die bekannten künstlichen Blasen bestehen aus einem elastischen Behälter, der in Aufweitungsrichtung vorgespannt ist. Ein Anschluß des Behälters ist mit einer Niere oder mit beiden Nieren verbunden, so daß der Behälter Urin aus der Niere ansaugt. Ein anderer Anschluß des Behälters ist mit der Harnröhre (Urethra) verbunden. Das Entleeren des Behälters erfolgt dadurch, daß der Behälter gegen die Kraft seiner Elastizität mit der Hand zusammengedrückt wird, wodurch die im Behälter befindliche Flüssigkeit in die Harnröhre hinein ausgedrückt wird. Die bekannten Blasen haben den Nachteil, daß der relativ große Behälter, der in der Regel ein flacher Rundbehälter ist, zu groß für den zwischen Rippen und Beckenschaufel zur Verfügung stehenden Platz ist und Unannehmlichkeiten beim Patienten bewirkt. Außerdem belastet der Behälter mit seiner Aufwölbung die Hautnaht an der Implantationsstelle.

Eine künstliche Blase muß selbstansaugend sein, d.h. sie muß Urin aus der Niere ansaugen, um die Funktion des Ureters zu ersetzen. Das Absaugen aus der Niere erfolgt normalerweise durch den Ureter mit peristaltischen Muskelbewegungen. Im Nierenbecken herrscht normalerweise ein geringer Überdruck, Voruntersuchungen haben jedoch gezeigt, daß vom Nierenparenchym auch geringe Unterdrücke akzeptiert werden. Wenn der Unterdruck von der Kunstblase erzeugt wird, ist es schwierig einen gleichmäßigen Unterdruck über alle Füllzustände der Kunstblase aufrechtzuerhalten. Um auch im nahezu gefüllten Zustand noch die erforderliche Saugwirkung zu erzeugen, sind die Kunstblasen üblicherweise so ausgelegt, daß sie im Leerzustand einen viel zu große Saugwirkung erzeugen, wodurch die Niere beschädigt werden kann.

Der Erfindung liegt die Aufgabe zugrunde, eine implantierbare künstliche Blase zu schaffen, die eine flache Bauweise ermöglicht und den Körper des Patienten weniger belastet.

Die Lösung dieser Aufgabe erfolgt erfindungsgemäß mit den im Patentanspruch 1 angegebenen Merkmalen.

Bei der erfindungsgemäßen Blase sind zwei Kammern vorgesehen, die durch ein Rückschlagventil verbunden sind. Beide Kammern sind in den Öffnungszustand vorgespannt, so daß sie eine Saugwirkung ausüben. Die Kammern sind nebeneinander angeordnet, wodurch sich das Kammervolumen räumlich verteilt. Diese Bauform ermöglicht eine bessere Anpassung an die Anatomie des Körpers, so daß die Blase zwischen Rippen und Beckenschaufel implantiert werden kann, ohne Belästigungen hervorzurufen. Ferner ergibt sich gegenüber den bekannten Blasen, die nur eine einzige Kammer aufweisen, eine flachere Bauweise bei gleichem Volumen. Als Folge hiervon wird die Hautnaht an der Implantationsstelle weniger belastet. Außerdem erhält die Kurve des Saugdrucks über dem Füllgrad der Blase einen gleichmäßigeren Verlauf. Daher reicht ein geringerer Saugdruck aus, durch den die Niere physiologisch nicht belastet wird.

Das Rückschlagventil zwischen den Kammern verhindert einen Rückfluß des Urins. Es ermöglicht es, den Inhalt der einlaßseitigen Kammer in die auslaßseitige Kammer hinein aus zudrücken.

Vorzugsweise sind die Saugwirkungen der beiden Kammern unterschiedlich, wobei die auslaßseitige Kammer die größte Saugwirkung hat. Das Rückschlagventil ist in Durchlaßrichtung vorgespannt, wobei es erst öffnet, wenn die Druckdifferenz zwischen beiden Kammern einen Grenzwert übersteigt. Dieser Grenzwert ist höher als die Druckdifferenz, die sich in den Kammern infolge unterschiedlicher Rückstellkräfte der Kammerwände einstellt. Wenn Flüssigkeit in die einlaufseitige Kammer einfließt und einen Teil des Kammervolumens füllt, übersteigt die Druckdifferenz den Grenzwert, so daß Flüssigkeit in die auslaßseitige Kammer hineinströmt, bis das Rückschlagventil wieder schließt. Auf diese Weise verringert sich der Saugdruck bzw. das Vakuum mit zunehmender Füllung der Blase nur relativ wenig.

Bei einer vorteilhaften Ausführungsform der Erfindung weisen die Kammern jeweils eine selbstrückstellende erste Wand und eine von der ersten Wand gespannte elastische Folienwand auf. Jede Kammer besteht also aus einer relativ dickwandigen biegeelastischen ersten Wand und einer Folienwand, die dehnungselastisch ist, jedoch praktisch keine Biegeelastizität hat.

Im folgenden wird ein Ausführungsbeispiel der Erfindung unter Bezugnahme auf die Zeichnungen näher erläutert.

Es zeigen:
- Fig. 1: einen Längsschnitt durch die künstliche Blase,
- Fig. 2: eine Draufsicht von Fig. 1, und
- Fig. 3: einen schematischen Verlauf des Unterdruck/Volumendiagramms der Blase.

Die dargestellte künstliche Blase besteht aus körperverträglichem elastischem Kunststoff, insbesondere aus Silikonmaterial. Sie weist einen generell 8-förmigen Grundriß auf, der durch zwei nebeneinanderliegende kreisrunde Kammern 10,11 gebildet wird. Die Kammer 10 weist eine konvex gewölbte Oberwand 12 auf, deren Wandstärke in der Mitte (im Scheitelbereich) am größten ist und zu den seitlichen Rändern hin abnimmt. Die Kammer 11 weist eine Oberwand 13 auf, die in gleicher Weise ausgebildet ist wie die Oberwand 12, wobei die Wandstärke jedoch größer ist, so daß in der Kammer 11 eine größere Saugwirkung erzeugt wird als in der Kammer 10.

Die Kammern 10,11 haben jeweils einen Boden, der durch eine Folienwand 14 bzw. 15 gebildet wird. Die Folienwände 14 und 15 gehen ineinander über und bestehen aus einer einstückigen Folie. Die Oberwände 12,13 sind an den umlaufenden Rändern mit den Folienwänden 14,15 verklebt oder verschweißt, so daß die Kammern 10,11 nach außen hermetisch abgeschlossen sind.

Die Kammer 10 weist im Scheitelpunkt der Oberwand 12 einen Anschluß 16 auf, an den ein externer Schlauch angeschlossen werden kann. Der Anschluß 16 ist mit einem Rückschlagventil 17 versehen, das über einen Schlauch 18 mit der Niere 19 verbunden wird und nur in die Kammer 10 hinein öffnet, in Gegenrichtung jedoch sperrt.

Die Oberwand 13 der Kammer 11 ist mit einem Anschluß 20 versehen, der mit einem Rückschlagventil 21 verbunden ist, welches nur in Auslaßrichtung öffnet, also aus der Kammer 11 heraus. Von dem Rückschlagventil 21 führt ein Schlauch 22 zu der (nicht dargestellten) Harnröhre.

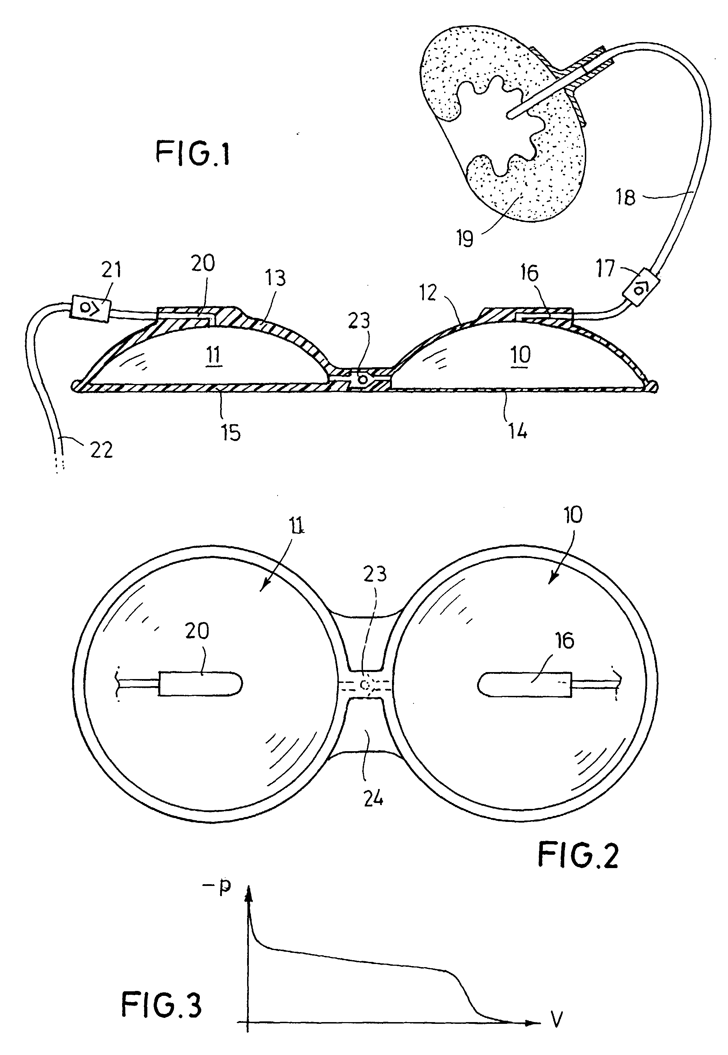

Bei der Darstellung von Fig. 2 sind die Rückschlagventile 17 und 21 in den jeweiligen Anschluß 16 bzw. 20 integriert. Die beiden Kammern 10,11 sind durch eine Verbindungslasche 24 verbunden. Sie bilden eine Blase in Form einer liegenden "8".

Zwischen den beiden Kammern 10 und 11 befindet sich ein Rückschlagventil 23, welches nur von der Kammer 10 zur Kammer 11 durchlässig ist, in Gegenrichtung jedoch sperrt. Es handelt sich um ein vorgespanntes Rückschlagventil, das erst dann öffnet, wenn die Druckdifferenz einen bestimmten Grenzwert übersteigt.

Im leeren Zustand der Kammern liegt die jeweilige Folienwand 14 bzw. 15 an der Innenseite der Oberwand 12 bzw. 13 an, wobei das Kammervolumen auf Null reduziert ist. Die Elastizität der Oberwand bestimmt die Rückstellkraft bzw. die Größe der Saugkraft, die von der Kammer ausgeübt wird, in Verbindung mit der Elastizität der Folienwand 14,15. Durch Wahl eines geeigneten Material für die Folienwand 14,15 kann unter Verwendung derselben Oberwand die Saugcharakteristik der Blase verändert werden.

Da die Oberwand 13 der zweiten Kammer 11 eine größere Wandstärke hat als die Oberwand 12 der ersten Kammer, wird von der zweiten Kammer eine größere Saugwirkung erzeugt als von der ersten Kammer. Die Druckdifferenz ist jedoch kleiner als der Grenzwert, bei dem das Rückschlagventil 23 öffnet.

Die erste Kammer 10 saugt mit der verringerten Saugkraft Flüssigkeit aus der Niere 19 an. Wenn sich die Kammer 10 zu einem Teil mit Flüssigkeit gefüllt hat, öffnet das Rückschlagventil 23, so daß ein Teil dieser Flüssgkeit in die zweite Kammer 11 gelangt. Auf diese Weise füllen sich beide Kammern nacheinander, wobei die Flüssigkeit zuerst immer in die Kammer 10 gelangt und dann einen Teil dieser Flüssigkeit in die Kammer 11 überführt wird. Wenn beide Kammern 10,11 gefüllt sind, erfolgt das Ausdrücken durch manuellen Druck auf die Kammer 11 bzw. durch Pumpwirkung. Durch Loslassen wird dann aus der Kammer 10 Flüssigkeit in die Kammer 11 nachgesaugt und anschließend durch Drücken wieder aus dieser herausgepreßt.

In Figur 3 ist schematisch der Verlauf des Unterdrucks "-" p über dem in der Blase enthaltenen Flüssigkeitsvolumen v dargestellt. Man erkennt, daß die Kurve relativ flach verläuft, d.h. daß der Saugdruck sich in Abhängigkeit vom Flüssigkeitsvolumen verhältnismäßig wenig ändert.

## Patentansprüche

1. Implantierbare künstliche Blase mit mindestens zwei zusammendrückbaren, in den Öffnungszustand vorgespannten Kammern (10,11), die jeweils einen Anschluß (16,20) aufweisen und durch ein Rückschlagventil (23) verbunden sind.

2. Blase nach Anspruch 1, dadurch gekennzeichnet, daß die Kammern (10,11) nebeneinander in einer 8-förmigen Konfiguration angeordnet sind.

3. Blase nach Anspruch 1 oder 2, dadurch gekennzeichnet, daß die Kammern (10,11) jeweils eine selbstrückstellende erste Wand (12,13) und eine von der ersten Wand gespannte elastische Folienwand (14,15) aufweisen.

4. Blase nach einem der Ansprüche 1-3, dadurch gekennzeichnet, daß das zwischen den Kammern (10,11) angeordnete Rückschlagventil (23) in Durchlaßrichtung vorgespannt ist und öffnet, wenn die Druckdifferenz einen Grenzwert übersteigt.

5. Blase nach einem der Ansprüche 1-4, dadurch gekennzeichnet, daß die stromab angeordnete Kammer (11) eine größere Saugwirkung hat als die stromauf angeordnete Kammer (10).
